# EUROPÄISCHE PATENTSCHRIFT

(11) **EP 2 462 906 B1**
(45) Veröffentlichungstag und Bekanntmachung des Hinweises auf die Patenterteilung: **31.07.2013**
(21) Anmeldenummer: 11405356.4
(22) Anmeldetag: 11.11.2011
(51) Int. Cl.: A61F 7/02

(54) **Temperiergerät für Thermobehandlungen**
Tempering device for thermal treatment
Appareil de régulation de température pour le traitement thermique

(30) Priorität: 13.12.2010 CH 20742010
(43) Veröffentlichungstag der Anmeldung: 13.06.2012
(73) Patentinhaber: Hilotherm Holding AG, 6317 Oberwil b. zug (CH)
(72) Erfinder: Titz, Peter, 6317 Oberwil bei Zug (CH)
(74) Vertreter: Clerc, Natalia

(56) Entgegenhaltungen:
- WO-A1-2010/088923
- WO-A2-2004/058111
- DE-A1- 19 535 640
- US-A1- 2009 227 924

## Beschreibung

### TECHNISCHES GEBIET

Die vorliegende Erfindung betrifft ein Temperiergerät oder Thermogerät für Thermobehandlungen gemäss Oberbegriff des Patentanspruchs 1.

### STAND DER TECHNIK

Das Auflegen von kühlen oder warmen Umschlägen oder Wickel auf die Haut ist ein altbekanntes und vielseitig einsetzbares Heilmittel. Es sind mittlerweile Temperiergeräte oder Thermogeräte bekannt, welche die Kühlung oder Erwärmung über eine längere Zeit aufrechterhalten können, da das erwärmende oder kühlende Medium in einem Vorratsbehälter im Gerät angeordnet ist. Das temperierende Medium wird über Schläuche zu einem Temperierkissen geführt, welches auf die Haut des Patienten aufgelegt wird. Die Temperierkissen weisen unterschiedliche Formen auf, wobei sie oft der entsprechenden Köperpartie des Patienten angepasst sind. Üblicherweise sind sie in Form von Manschetten ausgebildet. WO 97/21412 offenbart beispielsweise derartige Manschetten.

Die meisten bekannten Temperiergeräte weisen den Nachteil auf, dass sie die Temperatur im Temperierkissen nur innerhalb einer relativ grossen Bandbreite regeln können. Es ist jedoch mittlerweile bekannt, dass je nach Art der Erkrankung zu hohe oder zu tiefe Temperaturen nicht nur keine Linderung bringen sondern sogar noch schädigende Wirkung haben können. Der jeweils akzeptable und wirksame Temperaturbereich beträgt oft einige wenige Grad Celsius.

WO 2010/088923 beschreibt ein Temperiergerät gemäß Oberbegriff des Anspruchs 1, welches ein gradgenaues, d.h. genaues

Therapie- und Heilverfahren ermöglicht. Die Temperatur im angeschlossenen Kühl- bzw. Wärmekissen schwankt, wenn überhaupt, nur minimal, so dass einerseits die Behandlung des Patienten optimiert und Schäden vermieden werden können. Dieses Gerät wird insbesondere, jedoch nicht ausschliesslich, in der Orthopädie, der Unfallchirurgie, der Rheumatologie, der Dermatologie aber auch in der Mund-, Kiefer- und Gesichtschirurgie sowie im Sportbereich eingesetzt.

### DARSTELLUNG DER ERFINDUNG

Es ist eine Aufgabe der Erfindung, ein verbessertes Temperier- oder Thermogerät zu schaffen.

Diese Aufgabe löst ein Temperiergerät mit den Merkmalen des Anspruchs 1.

Das erfindungsgemässe Temperiergerät für Thermobehandlungen von Hautpartien des menschlichen und/oder tierischen Körpers weist ein Kühl- und oder Heizaggregat zur Kühlung oder Erwärmung eines Fluids, einen Thermo- oder Temperiertank zur Aufnahme dieses Fluids sowie Temperatursteuermittel zum Einstellen einer ersten Solltemperatur des Fluids auf. Der Temperiertank ist zwecks Kühlung und/oder Erwärmung der zu behandelnden Hautpartie mittels vom Fluid durchströmbaren Leitungen mit einem auf die zu behandelnde Hautpartie auflegbaren Temperierkissen verbindbar. Erfindungsgemäss umfassen die Steuermittel ein von einem Benützer betätigbares Eingabemittel, durch dessen Betätigung das Temperiergerät von einem Normalbetrieb in einen Schnellbetrieb wechselt, worauf die Temperatur des Fluids in einem Schnellverfahren von der ersten Solltemperatur auf eine zweite Temperatur wechselt.

Dieses Temperiergerät weist den Vorteil auf, dass bei speziellen Anwendungen, beispielsweise bei akuten Verletzungen bei einem Schlaganfall oder nach einer cardiopulmonalen Reanimation, sehr schnell gekühlt oder geheizt werden kann. Dies ist insbesondere dann wichtig, wenn das Gerät vorgängig zum Heizen eingesetzt wurde und nun möglichst schnell gekühlt werden soll. Der umgekehrte Fall ist auch möglich, d.h. zuerst Kühlung, dann Erwärmung. In einer bevorzugten Ausführungsform lässt sich zudem im Schnellbetrieb eine höhere bzw. tiefere Maximaltemperatur erreichen als im Normalbetrieb. Es hat sich gezeigt, dass im Vergleich zum Normalbetrieb eine Zieltemperatur im Schnellbetrieb in annähernd der halben oder einer noch kürzeren Zeit erreicht werden kann. In bevorzugten Ausführungsformen läst sich eine Temperatur im Bereich von 18°C bis 5°C in weniger als 1 Minute erreichen.

In einer einfachen Ausführungsform wird im Schnellbetrieb eine maximal erreichbare Temperatur angestrebt, ohne dass diese gradgenau angesteuert wird. Dies hat den Vorteil, dass sehr schnell eine grosse Temperaturänderung erzielt werden kann.

In einer bevorzugten Ausführungsform wird jedoch auch im Schnellbetrieb das Fluid, welches dem Temperierkissen zugeleitet wird, auf eine Temperatur reguliert, welche innerhalb eines relativ kleinen Bereichs liegt. Vorzugsweise liegt die akzeptierte Temperaturschwankung bei ±0.5°C.

Diese zweite im Schnellbetrieb erzielte Temperatur kann, muss aber nicht der Maximaltemperatur entsprechen. Sie kann auch einer oft verwendeten Temperatur entsprechen, wie sie beispielsweise in der Notfallmedizin oft verwendet wird. Diese zweite Temperatur lässt sich als zweite Solltemperatur mit den Temperatursteuermitteln des Geräts einstellen, wobei diese Einstellung dank des Schnellbetriebs nun relativ rasch erfolgen kann. Vorzugsweise ist die zu erzielende Temperatur bei Bedienung des Eingabemittels für den Schnellbetrieb bereits werkseitig vorgegeben und kann vom Benützer nicht gewählt werden. Es besteht jedoch auch die Möglichkeit, dass der Benützer eine im Schnellbetrieb zu erzielende Temperatur festlegen kann, beispielsweise bei Inbetriebnahme des Geräts. Diese möglichst genaue Einstellung der Temperatur auch im Schnellbetrieb hat den Vorteil, dass das Gerät für eine spezifische Notsituation optimal konfiguriert und einsetzbar ist. Vorzugsweise schaltet das Gerät bei Erreichen der Solltemperatur wieder auf Normalbetrieb um und versorgt anschliessend das Temperierkissen im Normalbetrieb mit dieser konstanten Temperatur.

In einer bevorzugten Ausführungsform ist das Eingabemittel zum Umschalten auf den Schnellbetrieb ein Betätigungsknopf oder ein Schalter, welcher vorzugsweise separat ausgebildet und somit von den übrigen Bedienungselementen getrennt ist. Vorzugsweise ist er optisch so gekennzeichnet, dass es auf einfache Art und Weise als Not- oder Expressbetätigungsmittel erkennbar und schnell betätigbar ist. Beispielsweise kann es mit der Farbe rot hervorgehoben sein. Dadurch wird auch in einer hektischen Notsituation keine Zeit eingebüsst.

Das erfindungsgemässe Gerät kann mit einem einzigen Eingabemittel zum Wechseln in den Schnellbetrieb ausgestattet sein. Es kann jedoch auch mehrere derartige Eingabemittel, beispielsweise mehrere Betätigungsknöpfe oder -schalter, aufweisen, wobei jedes Eingabemittel auf eine andere Temperatur wechselt. Dabei sind diese Temperaturen voneinander unterschiedlich. Dies erleichtert die Bedienung des Geräts in unterschiedlichen Situationen und Anwendungen.

Der Schnellbetrieb lässt sich auf unterschiedliche Art und Weise erreichen.

In einer ersten Ausführungsform ist ein zweites Kühl- oder Heizaggregat vorhanden, welches im Schnellbetrieb aktiviert wird. Dieses kann im Falle eines Tanks mit mehreren Kammern auch in einer anderen Kammer als das erste Kühlaggregat angeordnet sein. Vorzugsweise sind die Aggregate jedoch in derselben Kammer angeordnet. In einer Ausführungsform sind unterschiedliche Kühlaggregate vorhanden, beispielsweise ein erstes Aggregat mit einem Kompressor, welcher für den Normalbetrieb und evtl. auch für die Schnellkühlung eingesetzt wird, und ein zweites Aggregat in Form eines thermoelektrischen Kühlaggregats für die alleinige oder zusätzliche Schnellkühlung.

In einer bevorzugten Ausführungsform sind für den Schnellbetrieb Mittel vorhanden, um bei der Erzeugung von Kälte bzw. Wärme entstehende Prozesswärme bzw. -kälte in die Umgebung abzuführen, wobei diese Abfuhr im Schnellbetrieb schneller erfolgt als im Normalbetrieb. In einer einfachen Ausführungsform wird die Prozesswärme im Normalbetrieb nicht aktiv abgeführt; es lassen sich hierfür beispielsweise Kühlrippen verwenden. Vorzugsweise wird jedoch die Prozesswärme bzw. -kälte auch im Normalbetrieb aktiv abgeführt, wobei im Schnellbetrieb die Leistung des abführenden Mittels erhöht ist. Dank der erhöhten Abfuhr der Prozesswärme bzw. -kälte wird der Wirkungsgrad bzw. die Leistung des Kühl- bzw. Heizaggregats erhöht und somit eine schnellere Abkühlung bzw. Erwärmung des Fluids erreicht. Üblicherweise ist die Abfuhr der Prozesswärme vor allem bei Kühlapplikationen von Bedeutung. Geheizt wird üblicherweise über elektrisch betriebene Heizelemente, bei welchen keine Prozesskälte abzuführen ist.

In einer bevorzugten Ausführungsform ist das Mittel zur schnellen Abfuhr der Prozesswärme mindestens ein Lüfter. Unter Lüfter wird hier eine Vorrichtung verstanden, welche Luft von einem Ort an einen anderen Ort transportieren kann, also beispielsweise ein Ventilator.

Vorzugsweise weist das erfindungsgemässe Temperier- oder Thermogerät einen ersten Lüfter auf zur Abfuhr von Prozesswärme bzw. -kälte, welcher im Normalbetrieb einsetzbar ist, und einen zweiten Lüfter, welcher ausschliesslich im Schnellbetrieb einsetzbar ist und welcher das Mittel zur schnellen Abfuhr der Prozesswärme bildet. Je nach Ausführungsform ist der zweite Lüfter im Schnellbetrieb exklusiv oder zusätzlich zum ersten Lüfter einsetzbar. Wird er zusätzlich zum ersten Lüfter eingesetzt, können beide Lüfter einen relativ kleinen Wirkungsgrad aufweisen, so dass sie in den Abmessungen relativ klein und zudem kostengünstig sind. Dies ist insbesondere bei portablen Temperiergeräten von Vorteil. Vorzugsweise sind beide Lüfter identisch ausgebildet und weisen dieselbe Leistung auf. Sie können jedoch auch unterschiedlich dimensioniert sein, insbesondere in Bezug auf ihre Leistung. Des Weiteren können auch mehr als zwei Lüfter eingesetzt werden, welche gemeinsam oder in Kaskaden eingesetzt werden.

In einer bevorzugten Ausführungsform weist das Kühl- oder Heizaggregat des Temperiergeräts mindestens einen Wärmetauscher auf, welcher bei der Erzeugung von Kälte bzw. Wärme entstehende Prozesswärme bzw. -kälte in die Umgebung abführt. Vorzugsweise steht dieser mindestens eine Wärmetauscher mit dem Mittel zur schnellen Abfuhr der Prozesswärme in Wirkverbindung. Wird ein Lüfter als Abfuhrmittel verwendet, so ist der Wärmetauscher vom Lüfter mit Luft beaufschlagbar. Vorzugsweise ist der mindestens eine Wärmetauscher sowohl vom ersten wie auch vom zweiten Lüfter mit Luft beaufschlagbar. Derartige Wärmetauscher werden insbesondere bei Kühlaggregaten eingesetzt, bei denen die Kälte durch einen Kompressor oder durch thermoelektrische Elemente erzeugt wird. Vorzugsweise ist der Lüfter, welcher im Schnellbetrieb eingeschaltet ist, an der Ansaugseite angeordnet. Die Ansaugseite ist diejenige Seite, auf welcher die Luft von aussen angesaugt und dem Wärmetauscher zugeführt wird. Der erste, für den Normalbetrieb zuständige Lüfter ist vorzugsweise an der gegenüberliegenden Seite angeordnet.

In einer bevorzugten Ausführungsform ist das Eingabemittel mit einer Drehzahlsteuerung des Lüfters verbunden, wobei eine Betätigung des Eingabemittels eine Erhöhung der Drehzahl des Lüfters zur Folge hat. Diese Erhöhung kann zusätzlich zur Verwendung des zweiten Lüfters stattfinden oder alternativ dazu, insbesondere wenn nur ein einziger Lüfter verwendet wird. Im Falle von zwei Lüftern ist das Eingabemittel mit einer Drehzahlsteuerung mindestens eines der zwei Lüfter verbunden, wobei eine Betätigung des Eingabemittels eine Erhöhung der Drehzahl dieses Lüfters zur Folge hat. Die Erhöhung der Drehzahl des Lüfters hat insbesondere bei Verwendung mit einem zweiten Lüfter den Vorteil, dass die Prozesswärme bzw. kälte noch schneller abgeführt werden kann und somit die Leistung des Kühl- oder Heizaggregats noch mehr erhöht wird.

In einer Ausführungsform ist das Eingabemittels mit einer Steuerung der zwei Lüfter verbunden, wobei eine Betätigung des Eingabemittels zur Folge hat, dass beide Lüfter in ihrem maximalen Leistungsmodus betrieben sind. Dies optimiert die Abfuhr der Prozesswärme bzw. -kälte.

Das erfindungsgemässe Temperiergerät mit dem Schnellbetrieb lässt sich beispielsweise in einem Temperiergerät verwirklichen, wie es in der eingangs erwähnten WO 2010/088923 beschrieben ist. Eine Ausführungsform eines derartigen Temperiergeräts ist auf dem Markt unter dem Namen HILOTHERM Clinic bekannt. Das erfindungsgemässe Temperiergerät lässt sich jedoch auch auf andere Art und Weise verwirklichen.

Weitere Ausführungsformen sind in den abhängigen Ansprüchen angegeben.

### KURZE BESCHREIBUNG DER ZEICHNUNGEN

Eine bevorzugte Ausführungsform der Erfindung wird im Folgenden anhand der

Zeichnungen beschrieben, die lediglich zur Erläuterung dienen und nicht einschränkend auszulegen sind. In den Zeichnungen zeigen:
- Figur 1: einen Längsschnitt durch ein erfindungsgemässes Temperiergerät in einer schematischen Darstellung und
- Figur 2: eine Ansicht des Temperiergeräts gemäss Figur 1 von oben, ohne Deckel.

### BESCHREIBUNG BEVORZUGTER AUSFÜHRUNGSFORMEN

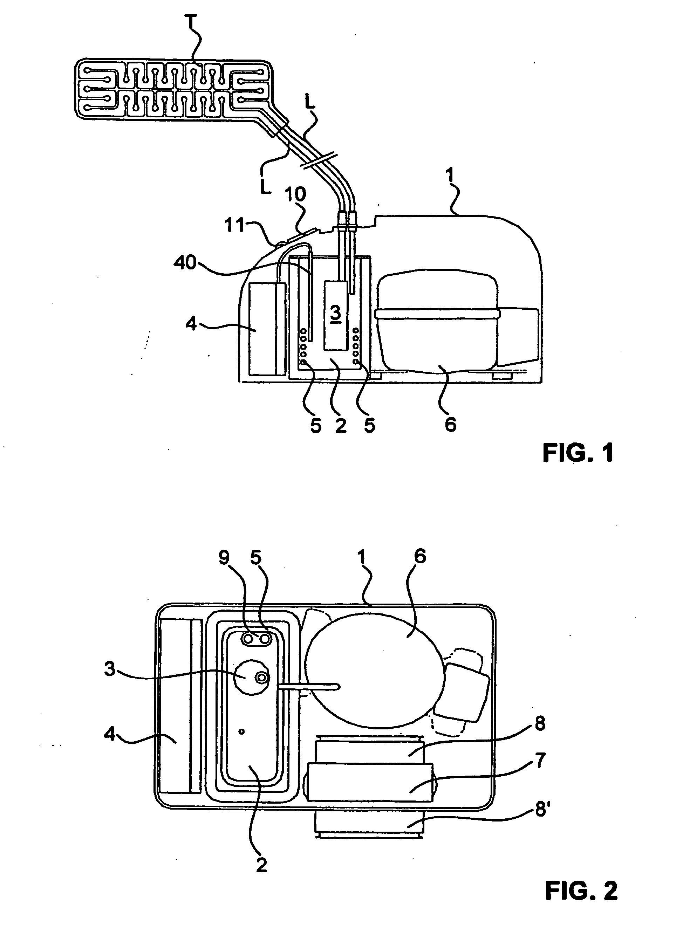

In den Figuren 1 und 2 ist ein erfindungsgemässes Thermo- oder Temperiergerät dargestellt.

In einem geschlossenen Gehäuse 1 ist ein Kühlaggregat 5, 6, 7, 8, 8', mindestens ein Temperiertank 2 zur Aufnahme des Temperierfluids, mindestens eine Pumpe 3, eine Elektronikeinheit 4 sowie mindestens ein Sensor 40 angeordnet.

In den Figuren nicht sichtbare Anschlüsse dienen zur Verbindung des Temperiergeräts 1 mit einem oder mehreren Temperierkissen T. Temperierfluid, welches im Tank 2 auf eine vorbestimmte Temperatur gekühlt ist, wird über mindestens eine Leitung L an das Temperierkissen T abgegeben, so dass es bei Auflage des Kissens T auf eine Körperpartie eines Patienten zur Heilung einsetzbar ist. Das Fluid fliesst über mindestens eine zweite Leitung L in das Gerät, vorzugsweise in den Temperiertank 2 zurück.

Zusätzlich oder alternativ zum Kühlaggregat kann ein Heizaggregat vorhanden sein. In diesem Beispiel ist hierfür zusätzlich eine Heizung 9 direkt im Temperiertank 2 angeordnet. Vorzugsweise ist die Heizung 9 ein elektrisches Widerstandsheizelement bekannter Art.

Im Temperiertank 2 sind Kühlwendel 5 des Kühlaggregats angeordnet, welche das Fluid im Tank 2 kühlen. Als Fluid wird vorzugsweise Wasser oder eine andere Kühlflüssigkeit eingesetzt. Der Temperiertank 2 ist wärmeisoliert ausgebildet, damit das im Tank 2 befindliche Temperier- oder Thermofluid nicht von der Umgebungstemperatur beeinflusst ist.

Der Tank 2 kann eine einzige Kammer aufweisen. Er kann jedoch auch, wie dies in WO 2010/088923 beschrieben ist, aus einer Temperierkammer und einer Mischkammer bestehen. Die Temperierkammer dient dabei zur Kühlung bzw. Erwärmung des Fluids mittels des Kühlaggregats bzw. der Heizung und somit als Vorratskammer für das temperierte Fluid. In der Mischkammer wird dann das vom Kühlkissen T zurückströmende Fluid mit Fluid aus der Vorratskammer gemischt und dem Kissen wieder zugeführt. Dadurch lässt sich die gewünschte Temperatur im Kissen T mit möglichst geringer bzw. ohne Gradabweichung aufrecht erhalten.

Je nachdem ob der Tank 2 ein- oder mehrkammrig ausgeführt ist, sind eine oder mehrere Pumpen 3 vorhanden, um Fluide von einer Kammer in die andere zu pumpen bzw. zu saugen und um das Kissen T mit dem Fluid zu durchströmen. Die Pumpe 3 ist vorzugsweise im Tank 2 angeordnet. Es kann sich beispielsweise um eine handelsübliche Tauchpumpe handeln. Die Pumpen lassen sich auch außerhalb des Tanks anordnen, um beispielsweise zu vermeiden, dass Abwärme der Pumpe in die kalte Flüssigkeit gelangt.

In diesem Beispiel ist ein Kompressions-Kühlaggregat eingesetzt. Es lassen sich jedoch auch andere Arten von Kühlaggregaten oder Kältequellen verwenden, welche Thermoelektrik, einen Adsorptionskälteprozess, oder CO₂ einsetzen. Die genannten Kühlwendel 5 sind mit einem Kompressor 6 verbunden, welcher ausserhalb des Tanks 2 angeordnet ist. Über mindestens einen Wärmetauscher 7 wird Prozesswärme abgeführt, welche im Kompressor beim Verdichten des Kältemittels entsteht.

Im Bereich des Wärmetauschers 7 ist ein erster Lüfter 8, insbesondere ein Ventilator, angeordnet, welcher Luft durch den Wärmetauscher 7 bläst bzw. saugt und so die Prozesswärme nach aussen abführt.

Erfindungsgemäss ist ein zweiter Lüfter 8' vorhanden, welcher ebenfalls mit dem Wärmetauscher 7 wirkverbunden ist. Auch er leitet einen Luftstrom durch den Wärmetauscher 7 und führt Prozesswärme ab. Vorzugsweise ist der zweite Lüfter 8' an der Ansaugseite des Wärmetauschers 7 angeordnet. Vorzugsweise befindet er sich auf der dem ersten Lüfter 8 gegenüberliegenden Seite des Wärmetauschers 7.

Am Gehäuse 1 sind nicht dargestellte Betätigungsmittel zur Betätigung des Geräts vorhanden. Diese Betätigungsmittel dienen beispielsweise zum Einschalten/Ausschalten des Geräts, zur Wahl einer an das Kühlkissen T anzulegende Temperatur, zur Wahl eines Therapieprogramms oder ähnlichem. Ein Display 10 zeigt zum Beispiel Funktionsweisen des Geräts, Wahlmöglichkeiten für den Benützer, Fehlermeldungen oder ähnliches auf. Die genannten Betätigungsmittel sind bekannte Mittel wie Schalter, Drehknöpfe oder Tasten.

Die Elektronikeinheit 4 ist mit diesen Betätigungsmitteln und dem Display 10 verbunden, nimmt Eingabebefehle auf und steuert das Gerät entsprechend. Hierfür ist die Elektronikeinheit mit den übrigen Elementen des Geräts, insbesondere dem mindestens einen Sensor 40, der mindestens einen Pumpe 3, dem Kühlaggregat 6 und den Lüftern 8, 8' verbunden. Die Elektronikeinheit wertet insbesondere Signale des mindestens einen Sensors 40, insbesondere eines das Temperierfluid überwachenden Temperatursensors 40, aus und steuert das Gerät entsprechend.

Bei Normalbetrieb steuert die Elektronikeinheit das Gerät so, dass Fluid mit der gewählten Temperatur vom Tank 2 zum Temperierkissen T gelangt, wobei die Leistung der Pumpe 3, des Kompressors 6 und des ersten Lüfters 8 entsprechend gesteuert werden. Wird eine neue Temperatur oder ein neues Therapieprogramm gewählt, so ändert sich die Steuerung entsprechend und es wird kontinuierlich auf die neue gewünschte Temperatur umgestellt.

Erfindungsgemäss ist nun ein Eingabemittel, hier ein Schnellbetrieb-Knopf 11, vorhanden, um das Gerät vom Normalbetrieb in einen Schnellbetrieb umschalten. Wird dieses Eingabemittel 11 betätigt, so wird in ein Schnellverfahren umgestellt, um die gewünschte neue Temperatur bzw. eine maximale mögliche Temperatur möglichst schnell zu erhalten. Dies kann auf unterschiedliche Weise erzielt werden. In diesem Beispiel wird hierfür der zweite Lüfter 8' aktiviert, damit die Prozesswärme schneller vom Wärmetauscher 7 abgeführt und somit die Leistung des Kühlaggregats erhöht wird. Falls der erste Lüfter 8 noch nicht mit maximaler Leistung, im Falle eines Ventilators mit maximaler Drehzahl, arbeitet, so wird vorzugsweise noch zusätzlich diese Leistung maximiert.

In diesem Beispiel wird auch im Schnellbetrieb die Temperatur des dem Temperierkissen T zugeführten Temperierfluids gemessen und überwacht. Vorzugweise wird in diesem Fall bei Erreichen der Temperatur ein akustisches und/oder Signal gegeben und/oder das Gerät schaltet automatisch auf Normalbetrieb um.

Anstelle eines einzigen Schnellbetriebs-Eingabemittels können auch mehrere derartige Eingabemittel vorhanden sein, welche das Gerät im Schnellbetrieb auf unterschiedliche Temperaturen bringen. In diesem Fall wählt die Steuerung je nach Temperaturwert oder Temperaturunterschied aus, ob ein oder zwei Lüfter eingesetzt werden und ob beide oder nur einer davon mit maximaler Leistung betrieben werden soll. Vorzugsweise werden jedoch im Schnellbetrieb immer beide Lüfter mit maximaler Leistung bzw. Drehzahl betrieben.

Das erfindungsgemässe Gerät ermöglicht eine optimierte Nutzung auch in Notsituationen und garantiert auch dort eine möglichst gradgenaue Temperaturapplikation.

### BEZUGSZEICHENLISTE

- 1: Gehäuse
- 10: Display
- 11: Schnellbetrieb-Knopf
- 2: Temperiertank
- 3: Pumpe
- 4: Elektronikeinheit
- 40: Temperatursensor
- 5: Kühlwendel
- 6: Kompressor
- 7: Wärmetauscher
- 8: erster Lüfter
- 8': zweiter Lüfter
- 9: Heizung
- T: Temperierkissen
- L: Verbindungsschlauch

## Patentansprüche

1. Temperiergerät für Thermobehandlungen von Hautpartien des menschlichen und/oder tierischen Körpers, wobei das Temperiergerät ein Kühl- und oder Heizaggregat (5, 6, 7, 8) zur Kühlung oder Erwärmung eines Fluids, einen Temperiertank (2) zur Aufnahme dieses Fluids sowie Temperatursteuermittel zum Einstellen einer ersten Solltemperatur des Fluids aufweist, wobei der Temperiertank (2) zwecks Kühlung und/oder Erwärmung der zu behandelnden Hautpartie mittels vom Fluid durchströmbaren Leitungen (L) mit einem auf die zu behandelnde Hautpartie auflegbaren Temperierkissen (T) verbindbar ist, **dadurch gekennzeichnet, dass** die Temperatursteuermittel ein von einem Benützer betätigbares Eingabemittel (11) umfassen, durch dessen Betätigung von einem Normalbetrieb in einen Schnellbetrieb des Temperiergerätts wechsel bar ist, worauf die Temperatur des Fluids in einem Schnellverfahren von der ersten Solltemperatur auf eine zweite Temperatur wechselt.

2. Temperiergerät nach Anspruch 1, wobei das Eingabemittel (11) ein Betätigungsknopf oder Schalter ist.

3. Temperiergerät nach einem der Ansprüche 1 oder 2, wobei die zweite Temperatur eine zweite Solltemperatur ist, welche von den Temperatursteuermitteln des Geräts einstellbar ist.

4. Temperiergerät nach einem der Ansprüche 1 bis 3, wobei mehr als ein Eingabemittel (11) vorhanden ist, welche in den Schnellbetrieb wechseln, wobei jedes Eingabemittel (11) auf eine zweite Temperatur wechselt, welche voneinander verschieden sind.

5. Temperiergerät nach einem der Ansprüche 1 bis 4, wobei das Kühl- oder Heizaggregat mindestens einen Wärmetauscher (7) aufweist, welcher bei der Erzeugung von Kälte bzw. Wärme entstehende Prozesswärme bzw. -kälte in die Umgebung abführt.

6. Temperiergerät nach einem der Ansprüche 1 bis 5, wobei Mittel (8, 8') vorhanden sind um bei der Erzeugung von Kälte bzw. Wärme entstehende Prozesswärme schneller in die Umgebung abzuführen.

7. Temperiergerät nach Anspruch 6, wobei das Mittel zur schnellen Abfuhr der Prozesswärme ein Lüfter (8, 8') ist.

8. Temperiergerät nach Anspruch 6, wobei es einen ersten Lüfter (8) aufweist zur Abfuhr von Prozesswärme bzw. -kälte, welche bei der Erzeugung von Kälte bzw. Wärme im Kühl- oder Heizaggregat entsteht, und welcher im Normalbetrieb einsetzbar ist, wobei ein zweiter Lüfter (8') vorhanden ist, welcher ausschliesslich im Schnellbetrieb einsetzbar ist und welcher das Mittel zur schnellen Abfuhr der Prozesswärme bildet.

9. Temperiergerät nach Anspruch 8, wobei der zweite Lüfter (8') im Schnellbetrieb zusätzlich zum ersten Lüfter (8) einsetzbar ist.

10. Temperiergerät nach den Ansprüchen 5 und 6, wobei der mindestens eine Wärmetauscher (7) mit dem Mittel (8, 8') zur schnellen Abfuhr der Prozesswärme in Wirkverbindung steht.

11. Temperiergerät nach den Ansprüchen 7 und 10, wobei der Wärmetauscher (7) vom Lüfter (8, 8') mit Luft beaufschlagbar ist.

12. Temperiergerät nach den Ansprüchen 8 und 10, wobei der mindestens eine Wärmetauscher (7) sowohl vom ersten wie auch vom zweiten Lüfter (8, 8') mit Luft beaufschlagbar ist.

13. Temperiergerät nach Anspruch 7, wobei das Eingabemittel (11) mit einer Drehzahlsteuerung (4) des Lüfters (8, 8') verbunden ist, wobei eine Betätigung des Eingabemittels (11) eine Erhöhung der Drehzahl des Lüfters (8, 8') zur Folge hat.

14. Temperiergerät nach einem der Ansprüche 8 oder 9, wobei das Eingabemittel (11) mit einer Drehzahlsteuerung (4) mindestens eines der zwei Lüfter (8, 8') verbunden ist, wobei eine Betätigung des Eingabemittels (11) eine Erhöhung der Drehzahl dieses Lüfters (8, 8') zur Folge hat.

15. Temperiergerät nach einem der Ansprüche 8 oder 9, wobei das Eingabemittel (11) mit einer Steuerung (4) der zwei Lüster (8, 8') verbunden ist, wobei eine Betätigung des Eingabemittels (11) zur Folge hat, dass beide Lüfter (8, 8') in ihrem maximalen Leistungsmodus betrieben sind.

## Claims

1. Temperature regulator for thermal treatments of areas of the skin of the human and/or animal body, wherein the temperature regulator has a cooling and/or heating unit (5, 6, 7, 8) for cooling or heating a fluid, a temperature regulator tank (2) for receiving this fluid, and temperature controls for setting a first target temperature of the fluid, wherein the temperature regulator tank (2), for the purpose of cooling and/or heating the area of skin to be treated, can be connected by means of lines (L), through which the fluid can flow, to a temperature control pad (T) that can be applied to the area of skin to be treated, **characterized in that** the temperature controls comprise an input means (11) that can be actuated by a user, the temperature regulator being able to be changed from a normal operating mode to a rapid operating mode by the actuation of the input means (11), whereupon the temperature of the fluid changes in a rapid method from the first target temperature to a second temperature.

2. Temperature regulator according to Claim 1, wherein the input means (11) is an actuating button or switch.

3. Temperature regulator according to either of Claims 1 and 2, wherein the second temperature is a second target temperature, which can be set by the temperature controls of the device.

4. Temperature regulator according to one of Claims 1 to 3, wherein there are more than one input means (11) changing to the rapid operating mode, wherein the input means (11) each change to second temperatures that are different from each other.

5. Temperature regulator according to one of Claims 1 to 4, wherein the cooling or heating unit has at least one heat exchanger (7) which, when cold or heat is generated, removes resulting process heat or cold into the environment.

6. Temperature regulator according to one of Claims 1 to 5, wherein means (8, 8') are present which, when cold or heat is generated, allow resulting process heat to be removed more rapidly into the environment.

7. Temperature regulator according to Claim 6, wherein the means for rapid removal of the process heat is a fan (8, 8').

8. Temperature regulator according to Claim 6, wherein it has a first fan (8) for removing process heat or cold that arises when cold or heat is generated in the cooling or heating unit, which first fan (8) can be used in the normal operating mode, wherein a second fan (8') is present which can be used exclusively in the rapid operating mode and which forms the means for rapid removal of the process heat.

9. Temperature regulator according to Claim 8, wherein the second fan (8') in the rapid operating mode can be used in addition to the first fan (8).

10. Temperature regulator according to Claims 5 and 6, wherein the at least one heat exchanger (7) is operatively connected to the means (8, 8') for rapid removal of the process heat.

11. Temperature regulator according to Claims 7 and 10, wherein the heat exchanger (7) can be exposed to air by the fan (8, 8').

12. Temperature regulator according to Claims 8 and 10, wherein the at least one heat exchanger (7) can be exposed to air both by the first fan (8) and also by the second fan (8').

13. Temperature regulator according to Claim 7, wherein the input means (11) is connected to a speed control (4) of the fan (8, 8'), wherein an actuation of the input means (11) has the effect of increasing the speed of the fan (8, 8').

14. Temperature regulator according to either of Claims 8 and 9, wherein the input means (11) is connected to a speed control (4) of at least one of the two fans (8, 8'), wherein an actuation of the input means (11) has the effect of increasing the speed of this fan (8, 8').

15. Temperature regulator according to either of Claims 8 and 9, wherein the input means (11) is connected to a control (4) of the two fans (8, 8'), wherein an actuation of the input means (11) has the effect that both fans (8, 8') are operated at their maximum power.

## Revendications

1. Thermorégulateur pour des traitements thermiques de parties de la peau du corps humain et/ou animal, le thermorégulateur présentant un groupe de refroidissement et/ou de chauffage (5, 6, 7, 8) pour refroidir ou chauffer un fluide, une cuve de thermorégulation (2) pour accueillir ce fluide ainsi que des moyens de contrôle de la température pour régler une première température de consigne du fluide, la cuve de thermorégulation (2) pouvant être reliée avec un coussin thermorégulateur (T) qui peut être appliqué sur la partie de la peau à traiter par le biais de conduites (L) dans lesquelles peuvent circuler le fluide en vue de refroidir et/ou de réchauffer la partie de la peau à traiter, **caractérisé en ce que** les moyens de contrôle de la température comprennent des moyens de saisie (11) pouvant être actionnés par un utilisateur, par l'actionnement desquels il est possible de basculer entre un mode de fonctionnement normal et un mode de fonctionnement rapide du thermorégulateur, après quoi la température du fluide, dans un procédé rapide, change de la première température de consigne à une deuxième température.

2. Thermorégulateur selon la revendication 1, avec lequel les moyens de saisie (11) sont un bouton d'actionnement ou un commutateur.

3. Thermorégulateur selon la revendication 1 ou 2, avec lequel la deuxième température est une deuxième température de consigne qui peut être réglée par les moyens de contrôle de la température de l'appareil.

4. Thermorégulateur selon l'une des revendications 1 à 3, avec lequel il existe plusieurs moyens de saisie (11) qui permettent de basculer dans le mode de fonctionnement rapide, les moyens de saisie (11) basculant respectivement sur des deuxièmes températures qui sont différentes les unes des autres.

5. Thermorégulateur selon l'une des revendications 1 à 4, avec lequel le groupe de refroidissement et/ou de chauffage présente au moins un échangeur thermique (7) qui, lors de la génération de froid ou de chaleur, évacue la chaleur ou le froid de procédé produit(e) dans l'environnement.

6. Thermorégulateur selon l'une des revendications 1 à 5, avec lequel il existe des moyens (8, 8') pour évacuer plus rapidement dans l'environnement la chaleur de procédé produite lors de la génération de froid ou de chaleur.

7. Thermorégulateur selon la revendication 6, avec lequel les moyens d'évacuation rapide de la chaleur de procédé sont des ventilateurs (8, 8').

8. Thermorégulateur selon la revendication 6, avec lequel il présente un premier ventilateur (8) pour évacuer la chaleur ou le froid de procédé qui se produit lors de la génération de froid ou de chaleur dans le groupe de refroidissement ou de chauffage et qui peut être utilisé en mode de fonctionnement normal, un deuxième ventilateur (8') étant présent, lequel peut exclusivement être utilisé en mode de fonctionnement rapide et lequel représente le moyen d'évacuation rapide de la chaleur de procédé.

9. Thermorégulateur selon la revendication 8, avec lequel le deuxième ventilateur (8') peut être utilisé en complément du premier ventilateur (8) en mode de fonctionnement rapide.

10. Thermorégulateur selon les revendications 5 et 6, avec lequel l'au moins un échangeur de chaleur (7) est en liaison active avec les moyens (8, 8') d'évacuation rapide de la chaleur de procédé.

11. Thermorégulateur selon les revendications 7 et 10, avec lequel l'échangeur de chaleur (7) peut être alimenté en air par le ventilateur (8, 8').

12. Thermorégulateur selon les revendications 8 et 10, avec lequel l'au moins un échangeur de chaleur (7) peut être alimenté en air par le premier ainsi que par le deuxième ventilateur (8, 8').

13. Thermorégulateur selon la revendication 7, avec lequel les moyens de saisie (11) sont reliés avec un contrôleur de vitesse de rotation (4) du ventilateur (8, 8'), un actionnement des moyens de saisie (11) ayant pour conséquence une augmentation de la vitesse de rotation du ventilateur (8, 8').

14. Thermorégulateur selon l'une des revendications 8 ou 9, avec lequel les moyens de saisie (11) sont reliés avec un contrôleur de vitesse de rotation (4) d'au moins l'un des deux ventilateurs (8, 8'), un actionnement des moyens de saisie (11) ayant pour conséquence une augmentation de la vitesse de rotation de ce ventilateur (8, 8').

15. Thermorégulateur selon l'une des revendications 8 ou 9, avec lequel les moyens de saisie (11) sont reliés avec un contrôleur (4) des deux ventilateurs (8, 8'), un actionnement des moyens de saisie (11) ayant pour conséquence que les deux ventilateurs (8, 8') fonctionnent dans leur mode de puissance maximale.
